(19)

Europäisches
Patentamt
European
Patent Office
Office européen
des brevets

(11) **EP 2 168 488 B1**

(12) **EUROPÄISCHE PATENTSCHRIFT**

(45) Veröffentlichungstag und Bekanntmachung des
Hinweises auf die Patenterteilung:
**13.02.2013 Patentblatt 2013/07**

(21) Anmeldenummer: **08017240.6**

(22) Anmeldetag: **30.09.2008**

(51) Int Cl.:
*A61B 6/02* (2006.01)     *A61B 6/03* (2006.01)
*G01N 23/04* (2006.01)

(54) **Röntgen-CT-System zur Röntgen-Phasenkontrast-und/oder Röntgen-Dunkelfeld-Bildgebung**

X-ray CT system for x-ray phase contrast and/or x-ray dark field imaging

Système de tomodensitométrie radiographique destiné à l'imagerie de radiographie à contrastes de phases et/ou de radiographie à champ obscur

(84) Benannte Vertragsstaaten:
**DE**

(43) Veröffentlichungstag der Anmeldung:
**31.03.2010 Patentblatt 2010/13**

(73) Patentinhaber:
• **Siemens Aktiengesellschaft
80333 München (DE)**
Benannte Vertragsstaaten:
**DE**
• **Paul Scherrer Institut
5232 Villigen PSI (CH)**
Benannte Vertragsstaaten:
**DE**

(72) Erfinder:
• **Hempel, Eckhard, Dr.
90865 Fürth (DE)**
• **Hoheisel, Martin, Dr.
91056 Erlangen (DE)**
• **Popescu, Stefan, Prof., Dr.
91056 Erlangen (DE)**
• **David, Christian, Dr.
79787 Lauchringen (DE)**
• **Donath, Tilman, Dr.
5200 Brugg (CH)**

• **Pfeiffer, Franz, Dr., Prof.
5200 Brugg (CH)**

(74) Vertreter: **Maier, Daniel Oliver
Siemens AG
Postfach 22 16 34
80506 München (DE)**

(56) Entgegenhaltungen:
**EP-A- 1 731 099**     **EP-A- 1 803 398**
**WO-A-2007/125833**     **US-A- 5 812 629**
**US-B2- 7 180 979**

• **WEITKAMP T ET AL: "Hard x-ray phase imaging and tomography with a grating interferometer" PROCEEDINGS OF THE SPIE, SPIE, BELLINGHAM, VA; US, Bd. 5535, Nr. 1, 1. Oktober 2004 (2004-10-01), Seiten 137-142, XP002397630 ISSN: 0277-786X**
• **MOMOSE A ET AL: "Biomedical imaging by Talbot-type X-ray phase tomography", PROCEEDINGS OF THE SPIE - THE INTERNATIONAL SOCIETY FOR OPTICAL ENGINEERING SPIE - THE INTERNATIONAL SOCIETY FOR OPTICAL ENGINEERING USA, vol. 6318, 7 September 2006 (2006-09-07), pages 63180T-1, ISSN: 0277-786X**

EP 2 168 488 B1

**Beschreibung**

Die Erfindung betrifft ein Röntgen-CT-System zur Röntgen-Phasenkontrast- und/oder Röntgen-Dunkelfeld-Bildgebung eines abgetasteten Untersuchungsobjektes mit mindestens einem, auf einer Gantry angeordneten Gitter-Interferometer, der mindestens eine Gitter-Interferometer aufweisend:

- eine erste Gitterstruktur, welche eine Vielzahl von streifenförmig und parallel angeordneten Röntgenemissionsmaxima und -minima aufweist, die eine erste Gitterperiode besitzen,
- eine zweite Gitterstruktur, die als Phasengitter einen teilweisen Phasenversatz einer durchtretenden Röntgenstrahlung bewirkt und eine zweite Gitterperiode aufweist,
- eine dritte Gitterstruktur mit einer dritten Gitterperiode, mit deren Hilfe eine relative Phasenverschiebungen benachbarter Röntgenstrahlen und/oder deren Streuanteile detektiert werden,
- wobei die drei Gitterstrukturen bezüglich ihrer Abstände untereinander und mindestens die erste und zweite Gitterstruktur bezüglich ihrer Gitterperioden die Talbot-Bedingungen erfüllen, und
- eine Vorrichtung zur wertemäßigen Bestimmung der Phase zwischen benachbarten Röntgenstrahlen und/oder zur wertemäßigen Bestimmung des räumlichen Intensitätsverlaufes je Detektorelement senkrecht zu den Streifen der Gitterstrukturen.

Derartige Röntgen-CT-System zur Röntgen-Phasenkontrast- und/oder Röntgen-Dunkelfeld-Bildgebung eines abgetasteten Untersuchungsobjektes sind allgemein bekannt. Beispielhaft wird auf die Druckschriften EP 1 731 099 A1, EP 1 803 398 A1 und DE 10 2006 017 290 A1 hingewiesen.

Der Einsatz von röntgenoptischen Gittern erlaubt einerseits die Aufnahme von Röntgenbildern im Phasenkontrast, welche zusätzliche Informationen über ein Untersuchungsobjekt liefern und/oder weniger Strahlendosis bei gleichem Bildkontrast ermöglichen. Andererseits besteht auch die Möglichkeit, dass zur Bildgebung nicht nur die Phaseninformation sondern auch die Amplitudeninformation gestreuter Strahlung verwendet wird. Hierdurch kann eine Bildgebung erzeugt werden, die ausschließlich auf den Streuanteilen der durch das Untersuchungsobjekt gebeugten Röntgenstrahlung basiert, also einer Kleinstwinkelstreuung. Hierdurch können sehr geringe Dichteunterschiede im Untersuchungsobjekt sehr hochauflösend dargestellt werden. Es wird diesbezüglich auf die Veröffentlichung von F. Pfeiffer et al.,"Hard X-ray dark-field imaging using a grating interferometer", Nature Materials 7, pp 134 - 137, verwiesen.

Um diese gewünschten Informationen eines mit inkohärenter Strahlung aus Röntgenröhren durchstrahlten Untersuchungsobjektes unter praktischen Bedingungen zu erhalten, müssen drei Gitterstrukturen verwendet werden, deren Perioden etwa im Bereich von 1 bis 100 Mikrometer liegen. Die Stege der mittleren Gitterstruktur - des Analysegitters - bestehen dabei aus phasenschiebendem Material und erzeugen einen Phasenschub von $\pi$ oder $\pi/2$ gemäß T. Weitkamp, et al.: Proc. SPIE 6318, Developments in X-Ray Tomography V (2006) p. 6318-28. Die beiden anderen Gitterstrukturen bestehen meist ebenfalls aus Gittern, wobei diese als Absorptionsgitter mit Stegen aus absorbierendem Material mit möglichst hoher Absorption ausgebildet sind.

Bisher wurde für Untersuchungen, bei denen tatsächlich an Detektoren pixelweise die Phasendifferenzen zwischen benachbarten Strahlen analytisch detektiert und bestimmt wurden bzw. bei denen zur Bildgebung nicht nur die Phaseninformation sondern auch die Amplitudeninformation analytisch bestimmt wurden, meist eine Anordnung gewählt, bei der der Abstand 1 zwischen erster und zweiter Gitterstruktur $G_0$ und $G_1$ grösser ist als der Abstand d zwischen der zweiten und dritten Gitterstruktur $G_1$ und $G_2$. Die Probe oder die Gantryöffnung war dabei zwischen der ersten und der zweiten Gitterstruktur $G_0$ und $G_1$ angeordnet. Dies hatte insbesondere zur Folge, dass für die entsprechenden Gitterperioden galt: $p_0 > p_1 > p_2$. Besonders die technische Realisierung der Gitterstruktur $G_2$ mit Absorberstrukturen erweist sich dabei problematisch, da es die kleinste Gitterperiode $p_2$ hat und die Gitterlinien eine hohe Absorption aufweisen müssen. Dies erfordert den Einsatz hochabsorbierender Materialien wie Gold. Gleichzeitig ist die Fläche von $G_2$ die grösste aller drei Gitter, was neben dem Herstellungsaufwand auch eine erhebliche Menge von teurem Gold erfordert.

In der Patentschrift US 5,812,629 wird in der Figur 5 ein CT-System mit einem Gitter-Interferometer gezeigt, bei dem das Untersuchungsobjekt zwischen dem zweiten und dritten Gitter angeordnet ist, wobei der Abstand zwischen den ersten beiden Gittern kleiner als der Abstand zwischen den letzten beiden Gittern ist. Allerdings wird in dieser Ausführung des CT-Systems keine wertemäßige Analyse des räumlichen Intensitätsverlaufes je Detektorelement durchgeführt und somit auch die Phase und Amplitude dieses Intensitätsverlaufes nicht analytisch bestimmt.

In der Patentschrift US 7,180,979 B2 wird eine Anordnung vorgeschlagen, bei der das Untersuchungsobjekt zwischen dem zweiten und dritten Gitter zu positioniert ist, allerdings wird in dieser Ausführung des CT-Systems keine wertemäßige Analyse des räumlichen Intensitätsverlaufes je Detektorelement durchgeführt und somit auch die Phase und Amplitude dieses Intensitätsverlaufes nicht analytisch bestimmt.

Weiterhin wird in der Patentanmeldung WO 2007/12533 A1 ein CT-System zur wertemäßigen Bestimmung von Phasenverschiebungen mit einem Talbot-Interferometer vorgeschlagen, bei dem die Gitterperioden in Strahlrichtung zunehmen, wobei das Untersuchungsobjekt zwischen der zweiten und dritten Gitterstruktur platziert werden kann, allerdings sind die dort vorgeschlagenen Verhältnisse der Gitterperioden zueinander und die Verhältnisse der Abstände zwischen den Gittern in der Praxis eines CT-Systems ungeeignet.

Es ist Aufgabe der Erfindung ein Röntgen-CT-System zur Röntgen-Phasenkontrast- und/oder Röntgen-Dunkelfeld-Bildgebung eines abgetasteten Untersuchungsobjektes mit mindestens einem auf einer Gantry angeordneten Gitter-Interferometer zu finden, welches im Rahmen der Talbot-Bedingungen für die verwendeten Gitterstrukturen geringere technische Anforderungen setzt und welches für den praktischen Betrieb zur Untersuchung von Objekten in der Größe menschlicher Patienten geeignet ist.

Diese Aufgabe wird durch die Merkmale des unabhängigen Patentanspruches 1 gelöst. Vorteilhafte Weiterbildungen der Erfindung sind Gegenstand untergeordneter Ansprüche.

Die Erfinder haben folgendes erkannt:

Die Bedingungen (=Talbot-Bedingungen) der Gitterperioden $p_0$, $p_1$, und $p_2$ der Gitterstrukturen $G_0$, $G_1$ und $G_2$ und die Abstände 1 zwischen $G_0$ und $G_1$ und d zwischen $G_1$ und $G_2$ können wie folgt beschrieben werden:

$$\frac{p_0}{p_2} = \frac{l}{d} \qquad\qquad [1]$$

Das Gitter $G_1$, welches von einer sphärischen Welle mit dem Radius 1 und der Wellenlänge $\lambda$ beleuchtet wird, erzeugt durch den Talbot-Effekt im Abstand TD' ein Interferenzmuster mit maximalem Kontrast. Es gilt:

$$TD' = \frac{l \cdot TD}{l - TD} \qquad\qquad [2]$$

wobei

$$TD = \frac{p_1^2}{8\lambda} \qquad\qquad [3]$$

der Talbotabstand für eine ebene Welle ist. Um optimalen Kontrast in der Messung zu erhalten, sollte $G_2$ in den Abstand TD' gesetzt werden, es gilt also d = TD'. Gleichzeitig sollte die Gitterperiode $p_2$ gleich der Periode des Interferenzmusters gewählt werden. Für dieses Interferenzmuster gilt:

$$p_2 = \frac{p_1}{2} \cdot \left(1 + \frac{d}{l}\right) \qquad\qquad [4]$$

Für eine gegebene Gesamtlänge des Messaufbaues s=1+d ergibt sich mit Hilfe der Gleichungen [2] und [3] eine erstaunlich einfache quadratische Gleichung für 1 mit:

$$l^2 - sl + sTD = 0 \qquad\qquad [5]$$

mit den Lösungen

$$l_{1,2} = \frac{s}{2} \pm \sqrt{\frac{s^2}{4} - sTD} = \frac{s}{2} \pm \sqrt{\frac{s^2}{4} - s\frac{p_1^2}{8\lambda}} \qquad\qquad [6]$$

Für $p_1 > \sqrt{2s\lambda}$ wird die Diskriminante kleiner null, es gibt also für solche Gitterperioden des Analysegitters $G_1$ keine Lösung innerhalb der Gesamtlänge s. $p_1 = \sqrt{2s\lambda}$ ergibt einen symmetrischen Aufbau bei dem 1=s/2=d und $p_0 = p_1 = p_2$ ist. Dieser Fall lässt allerdings keinen Platz für eine große Probe oder eine große Gantryöffnung in der Mitte zwischen Quelle und Detektor. Der Wurzelterm in Gleichung [6] beschreibt dabei den maximal möglichen Radius r einer Gantryöffnung mit:

$$r = \sqrt{\frac{s^2}{4} - s\frac{p_1^2}{8\lambda}} \ . \qquad\qquad [7]$$

Für $p_1 < \sqrt{2s\lambda}$ gibt es zwei Lösungen. Im bisher bekannten Stand der Technik wurde bei CT-Systemen, bei denen tatsächlich je Detektorelement analytische Werte der Phase zwischen benachbarten kohärenten Röntgenstrahlen bestimmt wurden, nur der Fall berücksichtigt bei dem 1 grösser als d ist und entsprechend das Untersuchungsobjekt zwischen der ersten und zweiten Gitterstruktur positioniert ist.

Entsprechend der Erkenntnis der Erfinder ist es entgegen der bisherigen Praxis jedoch besonders günstig einen Aufbau zu wählen, bei dem 1 kleiner als d ist. Hierdurch kann nämlich aufgrund der oben beschriebenen Talbot-Bedingungen erreicht werden, dass die Gitterperiode $p_2$ der dritten Gitterstruktur $G_2$ größer als die Gitterperiode $p_1$ der zweiten Gitterstruktur $G_1$ und diese wiederum größer als die Gitterperiode $p_2$ der zweiten Gitterstruktur $G_2$ ausfällt. Da geometrisch bedingt die erste Gitterstruktur $G_0$ in ihrer Fläche kleiner ausfällt als die der zweiten und die kleiner als die der dritten Gitterstruktur, ergibt sich ein wesentlich einfacherer Aufbau des Interferometers.

Eine solche Anordnung bringt im Vergleich zu der bisher verwendeten Anordnung folgende Vorteile mit sich:

1. Das Gitter mit der kleinsten Gitterperiode, welches entsprechend schwierig in der Herstellung ist, ist nun das $G_0$, welches eine viel kleinere Fläche hat als $G_2$. Dies reduziert Aufwand und Kosten.

2. $G_0$ kann durch eine entsprechend strukturierte Anode bzw. einen entsprechend strukturierten Elektronenstrahl auf einer Anode ersetzt werden, was möglicherweise einfacher zu realisieren ist als ein Gitter und sich leichter schrittweise oder kontinuierlich bewegen lässt.

3. Das Gitter $G_1$ hat zwar die gleiche Periode $p_1$, jedoch verkleinert sich seine Abmessung deutlich in Vergleich zur herkömmlichen Anordnung (dadurch ebenfalls Kostenreduzierung).

4. Das Gitter mit der grössten Fläche ist nun (nur) das Gitter $G_2$, die Anforderungen an die Mikrostrukturierung ist wesentlich geringer wegen der grossen Gitterperiode $P_2$.

5. Das Gitter $G_2$ kann wegen der gröberen Periode auch aus weniger stark absorbierendem Material als dem bisher verwendeten Gold gefertigt werden, da die Stege vergleichsweise problemlos dicker gemacht werden können. Die Verwendung z.B. von Blei statt Gold kann die Kosten deutlich senken.

6. $p_2$ kann so gross gewählt werden, dass es weggelassen und durch einen Streifen- oder Pixeldetektor mit entsprechender Periode ersetzt werden kann. Das vereinfacht den Aufbau und vermeidet, dass die Hälfte der hinter der Probe verfügbaren Röntgenphotonen in $G_2$ absorbiert wird.

Die Phasensensitivität der herkömmlichen und der hier vorgeschlagenen Anordnung ist nach momentanen Erkenntnissen gleich.

Entsprechend dieser Erkenntnis schlagen die Erfinder vor, ein an sich bekanntes Röntgen-CT-System zur Röntgen-Phasenkontrast- und/oder Röntgen-Dunkelfeld-Bildgebung eines abgetasteten trast- und/oder Röntgen-Dunkelfeld-Bildgebung eines abgetasteten Untersuchungsobjektes mit mindestens einem, auf einer Gantry angeordneten Gitter-Interferometer, zu verbessern, wobei das mindestens eine Gitter-Interferometer aufweist:

- eine erste Gitterstruktur, welche eine Vielzahl von streifenförmig und parallel angeordneten Röntgenemissionsmaxima und -minima aufweist, die eine erste Gitterperiode besitzen,

- eine zweite streifenförmige Gitterstruktur, die als Phasengitter einen teilweisen Phasenversatz einer durchtretenden Röntgenstrahlung bewirkt und eine zweite Gitterperiode aufweist,

- eine dritte streifenförmige Gitterstruktur mit einer dritten Gitterperiode, mit deren Hilfe eine relative Phasenverschiebungen benachbarter Röntgenstrahlen und/oder deren Streuanteile detektiert werden,

- wobei die drei Gitterstrukturen bezüglich ihrer Abstände untereinander und mindestens die erste und zweite Gitterstruktur bezüglich ihrer Gitterperioden die Talbot-Bedingungen erfüllen, und

- eine Vorrichtung zur wertemäßigen Bestimmung der Phase zwischen benachbarten Röntgenstrahlen und/oder zur wertemäßigen Bestimmung des räumlichen Intensitätsverlaufes je Detektorelement senkrecht zu den Streifen der Gitterstrukturen.

Die erfindungsgemäße Verbesserung besteht darin, dass die dritte Gitterstruktur eine Gitterperiode aufweist, die um einen Faktor 2 bis 5 größer ist als die Gitterperiode der ersten Gitterstruktur.

Weiterhin ist es zur Untersuchung großer Objekte günstig, wenn das mindestens eine Gitter-Interferometer einen Strahlengang aufweist, der in Richtung eines Rotationswinkels der Gantry eine Aufweitung von mindestens 30°, vorzugsweise von mindestens 35° bis 40°, aufweist.

Vorteilhaft kann das Untersuchungsobjekt zwischen der zweiten Gitterstruktur und der dritten Gitterstruktur positioniert werden, hierdurch kann sich ein relativ großes und zentrales Messfeld ergeben.

Vorteilhaft kann bei dem erfindungsgemäßen Röntgen-CT-System auch eine Ausdehnung der ersten Gitterstruktur in Umfangsrichtung der Gantry gewählt werden, die 1 bis 3 cm, vorzugsweise etwa 2 cm, beträgt.

Weiterhin kann die Ausdehnung der dritten Gitterstruktur in Richtung der größten Auffächerung der verwendeten Strahlung mindestens um den Faktor zwei größer sein als die Ausdehnung der zweiten Gitterstruktur in Richtung der größten Auffächerung der verwendeten Strahlung.

Bezüglich des Verhältnisses 1/d des Abstandes 1 zwischen der ersten und zweiten Gitterstruktur und dem Abstand d zwischen der zweiten und dritten Gitterstruktur wird vorgeschlagen, dass dieser kleiner 1 ist, vorzugsweise zwischen der Werten 1/d= 0,4 bis 0,2 liegt. Damit ist der Aufbau eines Gitter-Interferometers für ein CT-System möglich, der ein ausreichend großes Messfeld ermöglicht, welches sich im Drehzentrum einer Gantry befindet, wenn das Interferometer eingebaut ist.

Die erste Gitterstruktur kann in einer konventionellen Ausführung ein Quellengitter mit in Strahlrichtung vorgelagerten Fokus einer Röntgenquelle aufweisen. Damit kann auf bekannte Konstruktionen von Röntgenröhren zurückgegriffen werden, wobei lediglich im Bereich des Austrittsfensters der Röhre ein relativ kleines Absorptionsgitter als Quellengitter zu positionieren ist.

Alternativ zur Verwendung eines Quellengitters kann die erste Gitterstruktur auch durch auf einer Anode streifenförmig abwechselnd austretende Strahlungsmaxima und Strahlungsminima gebildet werden.

Zur Ausbildung solcher streifenförmiger Strahlungsmaxima und Strahlungsminima von Röntgenstrahlung sind unterschiedliche Methoden bekannt. Beispielsweise kann eine Anode verwendet werden, die eine inhomogen strukturierte Anodenoberfläche besitzt, wodurch die streifenförmig abwechselnd austretenden Strahlungsmaxima und Strahlungsminima entstehen. Eine solche Inhomogenität kann dadurch gebildet werden, dass die Anodenoberfläche streifenförmig angeordnete Erhebungen und/oder Vertiefungen aufweist. Es besteht jedoch auch die Möglichkeit auf der Anodenoberfläche streifenförmig Materialien mit unterschiedlicher Kernladungszahl anzuordnen. Auch eine Kombination der beiden letztgenannten Möglichkeiten ist möglich, indem in den Vertiefungen andere Materialien auf der Oberfläche vorliegen als auf relativen Erhebungen hierzu. Eine entsprechende Ausbildung ist in der Druckschrift EP 1 803 398 A1 gezeigt.

Eine weitere Möglichkeit streifenförmige Strahlungsmaxima und -minima auf einer Oberfläche zu erzeugen, besteht darin, eine auf elektro-magnetischer Basis arbeitende Ablenkungsvorrichtung eines Elektronenstrahls vorzusehen, die mit dem Elektronenstrahl die Anodenoberfläche streifenförmig abtastet und damit die streifenförmig abwechselnd austretenden Strahlungsmaxima und Strahlungsminima erzeugt. Auch diese Variante der Ausbildung ist in der Druckschrift EP 1 803 398 A1 gezeigt.

Zur wertemäßigen Analyse einer Phase zwischen benachbarten Röntgenstrahlen kann die dritte Gitterstruktur derart aufgebaut sein, dass sie mindestens ein Analysegitter mit danach angeordnetem ortsauflösenden Detektor mit einer Vielzahl von Detektorelementen aufweist. Hierbei kann weiterhin eine Vorrichtung zum kontrollierten Ortsversatz senkrecht zu ihren Gitterlinien und mit einer Ortsauflösung im Bereich der Periode der ersten Gitterstruktur vorgesehen werden. Alternativ zur Bewegung des Analysegitters kann auch die zweite Gitterstruktur eine Vorrichtung zum kontrollierten Ortsversatz senkrecht zu ihren Gitterlinien und mit einer Ortsauflösung im Bereich der Periode der ersten Gitterstruktur oder auch die dritte Gitterstruktur eine Vorrichtung zum kontrollierten Ortsversatz senkrecht zu ihren Gitterlinien und mit einer Ortsauflösung im Bereich der Periode der dritten Gitterstruktur aufweisen. Grundsätzlich besteht auch die Möglichkeit das Objekt selbst zu bewegen, allerdings erscheint dies zumindest bei einem Scan eines Patienten nicht praktikabel.

Anstelle der Bewegung von Gitterstrukturen kann auch eine dritte Gitterstruktur verwendet werden, die durch eine Vielzahl von pro detektiertem Röntgenstrahl streifenförmig ortsaufgelösten Detektorelementen gebildet wird, wie es beispielsweise in der Druckschrift DE 10 2006 017 290 A1 beschrieben wird.

Im Folgenden wird die Erfindung mit Hilfe der Figuren näher beschrieben, wobei nur die zum Verständnis der Erfindung notwendigen Merkmale dargestellt sind. Es werden folgende Bezugszeichen, Variablen und Kurzbezeichnungen verwendet: C1: Röntgen-CT-System; C2: Gantrygehäuse; C3: Gantryöffnung; C4: verschiebbare Patientenliege; C5: Patient; C6: Systemachse; C7: Steuer- und Recheneinheit; C8: Speicher; d: Abstand zwischen zweiten Gitter und dritten Gitter; D: Detektor; $E_i$: Detektorelemente; F: Fokus; $G_0$: Quellengitter; $G_1$: Phasengitter; $G_2$: Analysegitter; 1: Abstand zwischen erstem Gitter und zweitem Gitter; M: Messfeld; $p_0$: Gitterperiode der ersten Gitterstruktur; $p_1$: Gitterperiode der zweiten Gitterstruktur; $p_2$: Gitterperiode der dritten Gitterstruktur; $Prg_1$-$Prg_n$: Computer-Programme; r: Radius; s: Abstand zwischen erstem Gitter und dritten Gitter; S: Strahlkegel; $S_1$, $S_2$: benachbarte Röntgenstrahlen; $\alpha$: Aufweitung.

Es zeigen im Einzelnen:

FIG 1 ein Röntgen-CT-System mit erfindungsgemäßem Strahler-Detektor-System in 3D-Darstellung,

FIG 2 ein bekanntes, als Gitter-Interferometer aufgebautes, Strahler-Detektor-System eines CTs,

FIG 3 ein erfindungsgemäßes Strahler-Detektor-System und

FIG 4 eine Prinzipdarstellung des Messprinzips zur Röntgen-Phasenkontrast- und Röntgen-Dunkelfeld-Messung.

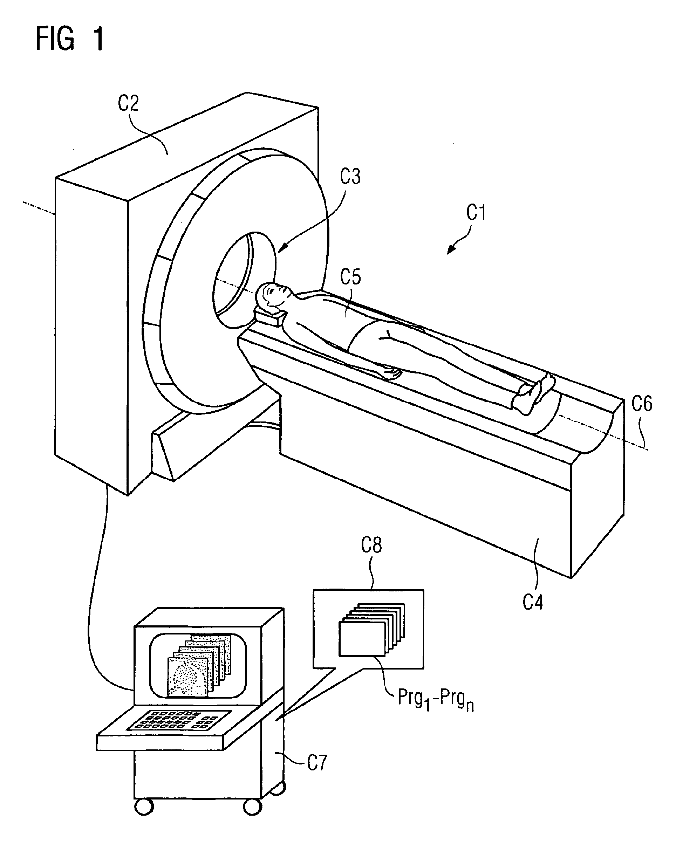

In der **Figur 1** ist ein erfindungsgemäßes Röntgen-CT-System C1 zur Röntgen-Phasenkontrast- und/oder Röntgen-Dunkelfeld-Bildgebung eines abgetasteten Untersuchungsobjektes mit mindestens einem, auf einer Gantry angeordneten Gitter-Interferometer, in 3D-Darstellung zu sehen. Das System besteht im Wesentlichen aus einem Gantrygehäuse C2, einer verschiebbaren Patientenliege C4 und einer Steuer- und Recheneinheit C7. Im Gantrygehäuse C2 ist eine Gantryöffnung C3 zu erkennen, die in ihrem Durchmesser etwa dem zweifachen Radius 2r des Messfeldes des oder der - hier nicht sichtbaren - Gitter-Interferometer auf der Gantry entspricht. Erfindungsgemäß sind die Gitterstrukturen der hier verwendeten Gitter-Interferometer so relativ zum Messfeld und damit zur Gantryöffnung C3 angeordnet, dass sich das Phasengitter auf der Quellenseite und die Analysegitterstruktur auf der Detektorseite befinden. Entsprechend den oben beschriebenen geometrischen Talbot-Bedingungen sind damit auch die Gitterstrukturen bezüglich ihrer Gitterperioden so ausgebildet, dass die relativ kleinste Gitterstruktur des Quellengitters $G_0$ auch die kleinste Gitterperiode $p_0$, die flächenmäßig größte Gitterstruktur des Anlaysegitters $G_2$ auch die größte Gitterperiode $p_2$ aufweist, wobei das Phasengitter $G_1$ bezüglich Ausdehnung und Gitterperiode dazwischen angeordnet ist. Hierdurch wird ein mit vertretbarem konstruktiven Aufwand realisierbares CT-System erreicht.

Zur Messung wird der Patient C5 mit Hilfe der verschiebbaren Patientenliege C4 bei rotierender Gantry entlang der Systemachse C6 sequenziell oder kontinuierlich durch das Messfeld geschoben, wobei eine Abtastung durch den einen oder mehrere mit der Gantry um die Systemachse C6 rotierende Gitter-Interferometer durchgeführt wird. Bei der Abtastung und der nachfolgenden Auswertung der Detektordaten werden quantitativ die Phasenunterschiede benachbarter kohärenter Röntgenstrahlen bestimmt und/oder es werden Dunkelfeld-CT-Aufnahmen aus Projektionen ähnlich den bekannten Dunkelfeldaufnahmen aus der Mikroskopie rekonstruiert. Hierzu werden zunächst aus einer Vielzahl von Projektionswinkeln die gebeugten Strahlungsanteile der abtastenden Röntgenstrahlung bestimmt. Anschließend werden diese projektiven Aufnahmen verwendet, um mit Hilfe an sich bekannter Rekonstruktionstechniken tomographische Bilddaten zu rekonstruieren, die volumenspezifische Kleinstwinkelstreuungen wiedergeben, wie es beispielsweise in F. Pfeiffer et al., "Hard X-ray dark-field imaging using a grating interferometer", Nature Materials 7, pp 134 - 137 gezeigt wird. Es wird darauf hingewiesen, dass für diese Aufnahmen eine genaue quantitative Kenntnis des räumlichen Intensitätsverlaufes senkrecht zur Streifenrichtung der Gitterstrukturen innerhalb jedes Röntgenstrahles notwendig ist.

Zur Durchführung der Steuerung, Messung und der Rekonstruktionen können hierbei die Computerprogramme $Prg_1$-$Prg_n$ dienen, die in einem Speicher C8 der Steuer- und Recheneinheit C7 abgelegt sind und die bei Bedarf abgerufen und ausgeführt werden können.

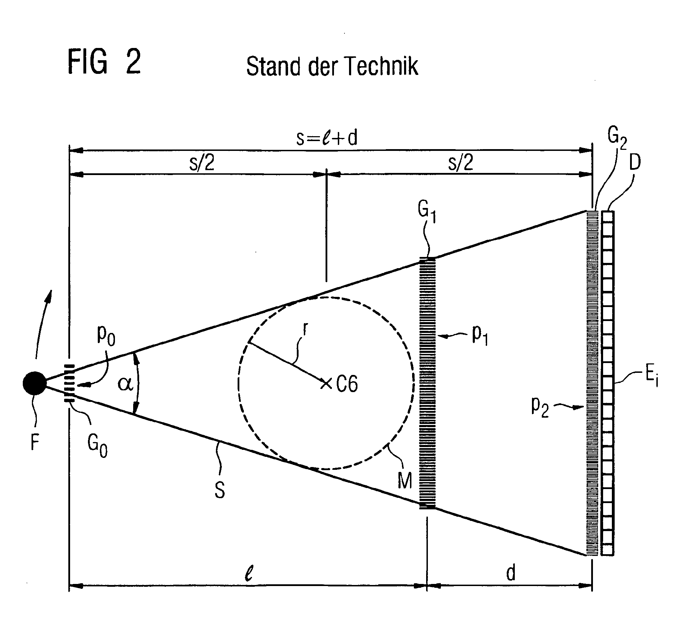

Die **Figur 2** zeigt den grundsätzlichen Aufbau eines Gitter-Interferometers in einer bisher verwendeten Anordnung mit seinen typischen Abmessungen. Links ist ein Fokus F einer Strahlungsquelle gezeigt, dem ein bezüglich der Gitterperiode $p_0$ relativ grob ausfallendes erstes Absorptionsgitter als Quellengitter $G_0$ folgt. Anschließend ist im Strahlkegel S, der eine große Aufweitung $\alpha$ aufweist, das Messfeld M angeordnet, welches etwa der Gantryöffnung C3 mit dem Radius r entspricht. Bei einem realistischen CT-System, bei dem das Messsystem und nicht das Untersuchungsobjekt rotiert, ist das Messfeld M konzentrisch zur Systemachse C6 der Gantry angeordnet. In Strahlrichtung folgt nun das Phasengitter $G_1$ mit einer kleineren Periode $p_1$ und darauf die dritte Gitterstruktur $G_2$ mit einer nochmals kleineren Gitterperiode $p_2$. In dem gezeigten Ausführungsbeispiel wird die Gitterstruktur $G_2$ durch das Analysegitter selbst und einen nachfolgenden Detektor D mit seinen Detektorelementen $E_i$ gebildet.

Wie aus dem gezeigten Beispiel zu erkennen ist, erfordert diese Anordnung auf der Detektorseite ein flächenmäßig sehr großes und gleichzeitig jedoch sehr feinstrukturiertes Absorptionsgitter als dritte Gitterstruktur. Eine solche Ausführung ist bei einer großen Aufweitung $\alpha$ jedoch nur sehr aufwendig zu realisieren.

Es wird daher erfindungsgemäß eine Ausführung vorgeschlagen, bei der die zweite Gitterstruktur $G_1$ auf die andere Seite des Messfeldes M verlegt wird und die Gitterperioden $p_0$,$p_1$ und $p_2$ in Strahlrichtung größer werden, so dass die flächenmäßig größte dritte Gitterstruktur $G_2$ auch am einfachsten herzustellen ist.

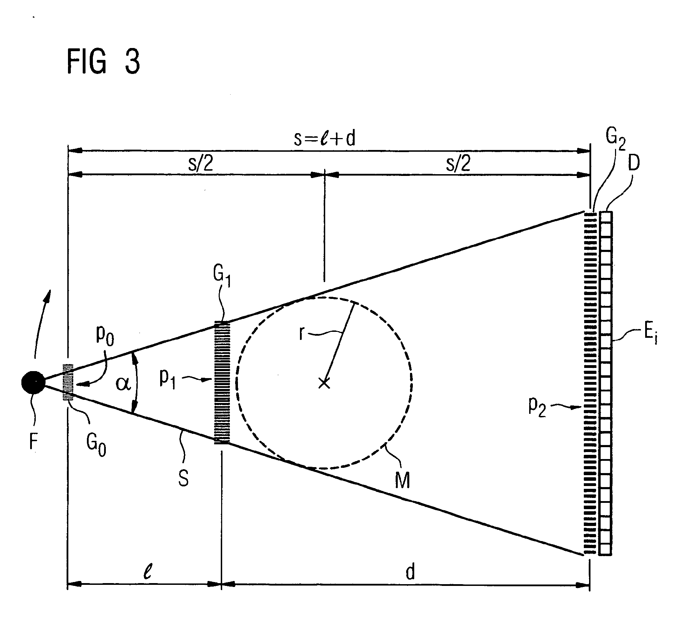

Solch ein beispielhafter Aufbau eines erfindungsgemäßen Gitter-Interferometers ist in der **Figur 3** dargestellt. Die charakteristischen Abstände s, 1 und d sind - ebenso wie in der Figur 2 - ebenfalls eingezeichnet. Typische Werte sind in der nachfolgenden Tabelle 1 gezeigt:

Tabelle 1

|  | s=l+d | 2r | $\alpha$ | l | d | E | $p_0$ | $p_1$ | $p_2$ |
|---|---|---|---|---|---|---|---|---|---|
| CT-Geometrie 1 | 1050mm | 700mm | 37° | 175mm | 875mm | 60keV | 2.95μm | 4.91μm | 14.7μm |
| CT-Geometrie 2 | 1050mm | 500mm | 27° | 275mm | 775mm | 60keV | 3.92μm | 5.79μm | 11.1μm |
| CT-Geometrie 3 | 1250mm | 700mm | 31° | 275mm | 975mm | 60keV | 3.81μm | 5.95μm | 13.5μm |

Wie aus der Tabelle 1 hervorgeht, kann ein in einem CT-System einzusetzendes Gitter-Interferometer so dimensioniert werden, dass einerseits ein ausreichend großes Messfeld mit einem Radius r von 700 mm zwischen der zweiten und dritten Gitterstruktur verbleibt und andererseits im Bereich der dritten Gitterstruktur für Gitterperioden von größer 10μm erreicht werden, die mit einem noch vertretbaren Aufwand erstellbar sind.

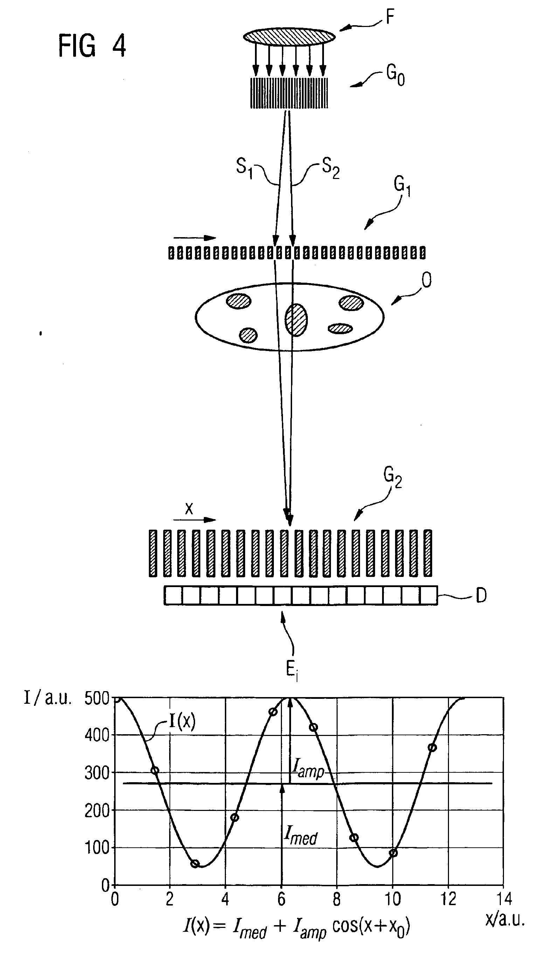

Zum besseren Verständnis des Messprinzips der erfindungsgemäßen CT-Systeme mit Gitter-Interferometern, wird noch auf die **Figur 4** verwiesen. Hier ist ein großflächiger Fokus F als Röntgenquelle und ein anschließend angeordnetes Quellengitter $G_0$, das hier die erste Gitterstruktur bildet, dargestellt. Hierdurch werden streifenweise quasikohärente Röntgenstrahlen an den Lücken des Quellengitters $G_0$ ausgestrahlt, während sie im Bereich der Absorption der Stege des Gitters weitgehend unterdrückt werden. Beispielhaft sind jeweils zwei benachbarte kohärente Röntgenstrahlen $S_1$ und $S_2$ gezeigt, die im Strahlenverlauf auf ein Phasengitter $G_1$ auftreffen, in dem eine Beugung der Strahlen stattfindet, so dass im Anschluss an das Phasengitter $G_1$ ein Interferenzmuster der gebeugten Röntgenstrahlung entsteht. Zur Sichtbarmachung dieses Interferenzmusters wird ein drittes Gitter, das Analysegitter, verwendet, welches einem Detektor D mit einer Vielzahl von Detektorelementen $E_i$ vorgelagert ist. Durch das Zusammenwirken der periodischen Interferenzen der Röntgenstrahlung mit dem Analysegitter $G_2$ ergibt sich hinter dem Analysegitter, je nach Position des Analysegitters $G_2$, eine Intensitätsschwankung der durchtretenden Röntgenstrahlung, die an den einzelnen Detektorelementen $E_i$ des Detektors D in Abhängigkeit von der Verschiebung des Gitters $G_2$ beziehungsweise auch von Verschiebungen der anderen vorgeschalteten Gitter gemessen werden kann. Das zu untersuchende Objekt ist zwischen dem Phasengitter $G_1$ und dem nachfolgendem Analysegitter $G_2$ positioniert.

Betracht man den Effekt der Verschiebung einer Gitterstruktur in x-Richtung auf die Intensitätsmessung eines Detektorelementes $E_i$, so ergibt sich ein Intensitätsverlauf I(x) in Abhängigkeit von der Gitterauslenkung x, wie er unten in der Figur 4 in Form einer Sinusschwingung gezeigt ist. Es gilt dabei:

$$I(x) = I_{med} + I_{amp} \cos(x + x_0)$$

Der Verlauf dieser Kurve lässt sich durch die Angabe des Mittelwertes $I_{med}$, die Angabe der Auslenkungsamplitude $I_{amp}$ und der Phase $x_0$, mit der die sinusförmige Auslenkung verläuft, vollständig beschreiben.

Im erfindungsgemäßen CT-System wird dieser Verlauf der Intensitätskurve in Abhängigkeit von der Verschiebung eines Gitters und Messung der Strahlungsintensität in Abhängigkeit von den Gitterpositionen wertemäßig ermittelt. Aus der Kenntnis dieses Verlaufes können dann für die Phasenkontrast-Bildgebung die Phasen $x_0$ ausgewertet werden oder für eine Dunkelfeld-Bildgebung die Mittelwerte $I_{med}$ und die Auslenkungsamplitude $I_{amp}$ in bekannter Weise ausgewertet werden.

**Patentansprüche**

1. Röntgen-CT-System (C1) zur Röntgen-Phasenkontrast- und/oder Röntgen-Dunkelfeld-Bildgebung eines abgetasteten Untersuchungsobjektes (C5) mit mindestens einem, auf einer Gantry angeordneten Gitter-Interferometer, der mindestens eine Gitter-Interferometer aufweisend:

　　1.1. eine erste Gitterstruktur ($G_0$), welche eine Vielzahl von streifenförmig und parallel angeordneten Röntgenemissionsmaxima und -minima aufweist, die eine erste Gitterperiode ($p_0$) besitzen,
　　1.2. eine zweite streifenförmige Gitterstruktur ($G_1$), die als Phasengitter einen teilweisen Phasenversatz einer durchtretenden Röntgenstrahlung bewirkt und eine zweite Gitterperiode ($p_1$) aufweist,

1.3. eine dritte streifenförmige Gitterstruktur ($G_2$) mit einer dritten Gitterperiode ($p_2$), mit deren Hilfe eine relative Phasenverschiebungen benachbarter Röntgenstrahlen ($S_1$, $S_2$) und/oder deren Streuanteile detektiert werden,

1.4. wobei die drei Gitterstrukturen ($G_0$, $G_1$, $G_2$) bezüglich ihrer Abstände (1, d) untereinander und mindestens die erste und zweite Gitterstruktur ($G_0$, $G_1$) bezüglich ihrer Gitterperioden ($p_0$, $p_1$) die Talbot-Bedingungen erfüllen, und

1.5. eine Vorrichtung zur wertemäßigen Bestimmung der Phase zwischen benachbarten Röntgenstrahlen und/ oder zur wertemäßigen Bestimmung des räumlichen Intensitätsverlaufes je Detektorelement senkrecht zu den Streifen der Gitterstrukturen,

**dadurch gekennzeichnet, dass**

1.6. die dritte Gitterstruktur ($G_2$) eine Gitterperiode ($p_2$) aufweist, die um einen Faktor 2 bis 5 größer ist als die Gitterperiode ($p_0$) der ersten Gitterstruktur ($G_0$).

2. Röntgen-CT-System (C1) gemäß dem voranstehenden Patentanspruch 1, **dadurch gekennzeichnet, dass** das mindestens eine Gitter-Interferometer einen Strahlengang aufweist, der in Richtung eines Rotationswinkels der Gantry eine Aufweitung ($\alpha$) von mindestens 30° aufweist.

3. Röntgen-CT-System (C1) gemäß einem der voranstehenden Patentansprüche 1 oder 2, **dadurch gekenn - zeichnet**, dass das mindestens eine Gitter-Interferometer einen Strahlengang aufweist, der in Richtung eines Rotationswinkels der Gantry eine Aufweitung ($\alpha$) von mindestens 35° bis 40° aufweist

4. Röntgen-CT-System (C1) gemäß einem der voranstehenden Patentansprüche 1 bis 3, **dadurch gekennzeichnet, dass** das Untersuchungsobjekt zwischen der zweiten Gitterstruktur ($G_1$) und der dritten Gitterstruktur ($G_2$) positioniert ist.

5. Röntgen-CT-System (C1) gemäß einem der voranstehenden Patentansprüche 1 bis 4, **dadurch gekennzeichnet, dass** die Ausdehnung der ersten Gitterstruktur ($G_0$) in Umfangsrichtung der Gantry 1 bis 3 cm beträgt.

6. Röntgen-CT-System (C1) gemäß einem der voranstehenden Patentansprüche 1 bis 5, **dadurch gekennzeichnet, dass** das die Ausdehnung der dritten Gitterstruktur ($G_2$) in Richtung der größten Auffächerung der verwendeten Strahlung mindestens um den Faktor zwei größer ist als die Ausdehnung der zweiten Gitterstruktur ($G_1$) in Richtung der größten Auffächerung der verwendeten Strahlung.

7. Röntgen-CT-System (C1) gemäß einem der voranstehenden Patentansprüche 1 bis 6, **dadurch gekennzeichnet, dass** das Verhältnis (l/d) des Abstandes (1) zwischen der ersten und zweiten Gitterstruktur ($G_0$, $G_1$) und dem Abstand zwischen der zweiten und dritten Gitterstruktur ($G_1$, $G_2$) kleiner 1 ist.

8. Röntgen-CT-System (C1) gemäß einem der voranstehenden Patentansprüche 1 bis 6, **dadurch gekennzeichnet, dass** das Verhältnis (l/d) des Abstandes (1) zwischen der ersten und zweiten Gitterstruktur ($G_0$, $G_1$) und dem Abstand zwischen der zweiten und dritten Gitterstruktur ($G_1$, $G_2$) zwischen der Werten l/d= 0,5 bis 0,1 liegt.

9. Röntgen-CT-System (C1) gemäß einem der voranstehenden Patentansprüche 1 bis 8, **dadurch gekennzeichnet, dass** die erste Gitterstruktur ($G_0$) ein Quellengitter ($G_0$) mit in Strahlrichtung vorgelagerten Fokus (F) einer Röntgenquelle aufweist.

10. Röntgen-CT-System (C1) gemäß einem der voranstehenden Patentansprüche 1 bis 8, **dadurch gekennzeichhnet**, dass die erste Gitterstruktur ($G_0$) durch auf einer Anode streifenförmig abwechselnd austretende Strahlungsmaxima und Strahlungsminima gebildet wird.

11. Röntgen-CT-System (C1) gemäß dem voranstehenden Patentanspruch 10, **dadurch gekennzeichnet, dass** die Anode eine inhomogen strukturierte Anodenoberfläche aufweist, wodurch die streifenförmig abwechselnd austretenden Strahlungsmaxima und Strahlungsminima entstehen.

12. Röntgen-CT-System (C1) gemäß dem voranstehenden Patentanspruch 11, **dadurch gekennzeichnet, dass** die Anodenoberfläche streifenförmig angeordnete Erhebungen und/oder Vertiefungen aufweist.

13. Röntgen-CT-System (C1) gemäß dem voranstehenden Patentanspruch 11, **dadurch gekennzeichnet, dass** die Anodenoberfläche streifenförmig angeordnete Materialien mit unterschiedlicher Kernladungszahl aufweist.

**14.** Röntgen-CT-System (C1) gemäß dem voranstehenden Patentanspruch 10, **dadurch gekennzeichnet, dass** eine auf elektro-magnetischer Basis arbeitende Ablenkungsvorrichtung eines Elektronenstrahls vorgesehen ist, die mit dem Elektronenstrahl die Anodenoberfläche streifenförmig abtastet und damit die streifenförmig abwechselnd austretenden Strahlungsmaxima und Strahlungsminima erzeugt.

**15.** Röntgen-CT-System (C1) gemäß einem der voranstehenden Patentansprüche 1 bis 14, **dadurch gekennzeichnet, dass** die dritte Gitterstruktur ($G_2$) mindestens ein Analysegitter mit danach angeordnetem ortsauflösenden Detektor (D) mit einer Vielzahl von Detektorelementen ($E_i$) aufweist.

**16.** Röntgen-CT-System (C1) gemäß einem der voranstehenden Patentansprüche 1 bis 15, **dadurch gekennzeichnet, dass** die erste Gitterstruktur ($G_0$) eine Vorrichtung zum kontrollierten Ortsversatz senkrecht zu ihren Gitterlinien und mit einer Ortsauflösung im Bereich der Periode ($p_0$) der ersten Gitterstruktur ($G_0$) aufweist.

**17.** Röntgen-CT-System (C1) gemäß einem der voranstehenden Patentansprüche 1 bis 15, **dadurch gekennzeichnet, dass** die zweite Gitterstruktur ($G_1$) eine Vorrichtung zum kontrollierten Ortsversatz senkrecht zu ihren Gitterlinien und mit einer Ortsauflösung im Bereich der Periode ($p_1$) der zweiten Gitterstruktur ($G_1$) aufweist.

**18.** Röntgen-CT-System (C1) gemäß einem der voranstehenden Patentansprüche 1 bis 15, **dadurch gekennzeichnet, dass** die dritte Gitterstruktur ($G_2$) eine Vorrichtung zum kontrollierten Ortsversatz senkrecht zu ihren Gitterlinien und mit einer Ortsauflösung im Bereich der Periode ($p_2$) der dritten Gitterstruktur ($G_2$) aufweist.

**19.** Röntgen-CT-System gemäß einem der voranstehenden Patentansprüche 1 bis 14, **dadurch gekennzeichnet, dass** die dritte Gitterstruktur ($G_2$) durch eine Vielzahl von pro detektiertem Röntgenstrahl streifenförmig ortsaufgelösten Detektorelementen ($E_i$) gebildet wird.

**Claims**

**1.** X-ray CT system (C1) for X-ray phase contrast and/or X-ray dark field imaging of a scanned examination subject (C5) comprising at least one grating interferometer arranged on a gantry, said at least one grating interferometer comprising:

1.1. a first grating structure ($G_0$) having a plurality of band-shaped X-ray emission maxima and minima arranged in parallel, said maxima and minima exhibiting a first grating period ($p_0$),
1.2. a second band-shaped grating structure ($G_1$) that produces, as a phase grating, a partial phase offset of X-ray radiation passing therethrough and that exhibits a second grating period ($p_1$),
1.3. a third band-shaped grating structure ($G_2$) with a third grating period ($p_2$), with which relative phase shifts of adjacent X-rays ($S_1$, $S_2$) and/or the scatter components thereof are detected,
1.4. wherein the three grating structures ($G_0$, $G_1$, $G_2$) with regard to the distances (1, d) from one another, and at least the first and second grating structures ($G_0$, $G_1$) with regard to their grating periods ($p_0$, $p_1$) satisfy the Talbot conditions, and
1.5. a device configured for value-based determination of the phase between adjacent X-rays and/or for value-based determination of the spatial intensity curve per detector element perpendicular to the bands of the grating structures,
**characterised in that**
1.6. the third grating structure ($G_2$) has a grating period ($p_2$) that is larger by a factor of 2 to 5 than the grating period ($p_0$) of the first grating structure ($G_0$).

**2.** X-ray CT system (C1) according to preceding claim 1, **characterised in that** the at least one grating interferometer has a beam path that, in the direction of a rotation angle of the gantry, exhibits a divergence ($\alpha$) of at least 30°.

**3.** X-ray CT system (C1) according to preceding claim 1 or 2, **characterised in that** the at least one grating interferometer has a beam path that, in the direction of a rotation angle of the gantry, exhibits a divergence ($\alpha$) of at least 35° to 40°.

**4.** X-ray CT system (C1) according to one of the preceding claims 1 to 3, **characterised in that** the examination subject is placed between the second grating structure ($G_1$) and the third grating structure ($G_2$).

5. X-ray CT system (C1) according to one of the preceding claims 1 to 4, **characterised in that** the dimension of the first grating structure ($G_0$) in a circumferential direction of the gantry is 1 to 3 cm.

6. X-ray CT system (C1) according to one of the preceding claims 1 to 5, **characterised in that** the dimension of the third grating structure ($G_2$) in a direction of greatest divergence of the radiation used is greater by at least a factor of two than the dimension of the second grating structure ($G_1$) in the direction of greatest divergence of the radiation used.

7. X-ray CT system (C1) according to one of the preceding claims 1 to 6, **characterised in that** the ratio (l/d) of the distance (1) between the first and second grating structures ($G_0$, $G_1$) and the distance between the second and third grating structures ($G_1$, $G_2$) is smaller than 1.

8. X-ray CT system (C1) according to one of the preceding claims 1 to 6, **characterised in that** the ratio (l/d) of the distance (1) between the first and second grating structures ($G_0$, $G_1$) and the distance between the second and third grating structures (G1, G2) is between the values l/d= 0.5 to 0.1.

9. X-ray CT system (C1) according to one of the preceding claims 1 to 8, **characterised in that** the first grating structure ($G_0$) has a source grating ($G_0$) with the focus (F) of an X-ray source situated upstream therefrom in the beam direction.

10. X-ray CT system (C1) according to one of the preceding claims 1 to 8, **characterised in that** the first grating structure ($G_0$) is formed by radiation maxima and radiation minima alternatingly emitted in bands at an anode.

11. X-ray CT system (C1) according to preceding claim 10, **characterised in that** the anode has an inhomogeneously structured anode surface, at which the alternatingly emitted band-shaped radiation maxima and radiation minima bands are created.

12. X-ray CT system (C1) according to preceding claim 11, **characterised in that** the anode surface comprises elevations and/or depressions arranged in bands.

13. X-ray CT system (C1) according to preceding claim 11, **characterised in that** the anode surface is comprised of materials with different atomic number arranged in bands.

14. X-ray CT system (C1) according to preceding claim 10, **characterised in that** a deflection device for an electron beam is provided that operates on an electro-magnetic basis, which deflection device scans the surface of the anode in a band-shaped manner with the electron beam and generates the alternatingly emitted radiation maxima and radiation minima in bands.

15. X-ray CT system (C1) according to one of the preceding claims 1 to 14, **characterised in that** the third grating structure ($G_2$) has at least one analysis grating with a subsequently arranged, spatially resolving detector (D) comprising a plurality of detector elements ($E_i$).

16. X-ray CT system (C1) according to one of the preceding claims 1 to 15, **characterised in that** the first grating structure ($G_0$) has a device for monitored spatial offset perpendicular to the grating lines thereof and with a spatial resolution approximating the period ($p_0$) of the first grating structure ($G_0$).

17. X-ray CT system (C1) according to one of the preceding claims 1 to 15, **characterised in that** the second grating structure ($G_1$) has a device for monitored spatial offset perpendicular to the grating lines thereof and with a spatial resolution approximating the period ($p_1$) of the second grating structure ($G_1$).

18. X-ray CT system (C1) according to one of the preceding claims 1 to 15, **characterised in that** the third grating structure ($G_2$) has a device for monitored spatial offset perpendicular to the grating lines thereof and with a spatial resolution approximating the period ($p_2$) of the third grating structure ($G_2$).

19. X-ray CT system (C1) according to one of the preceding claims 1 to 14, **characterised in that** the third grating structure ($G_2$) is formed by a plurality of band-shaped, spatially resolved detector elements ($E_i$) per detected X-ray beam.

**Revendications**

1. Système de tomodensitométrie par rayons X (C1) destiné à l'imagerie à contraste de phase par rayons X et/ou à fond noir par rayons X d'un objet à examiner (C5) balayé, comprenant au moins un interféromètre à réseau disposé sur un portique, ledit au moins un interféromètre à réseau comprenant :

   1.1. une première structure de réseau ($G_0$), comprenant une pluralité de maxima et de minima d'émission de rayons X disposés en forme de bande et en parallèle et possédant une première période de réseau ($p_0$),
   1.2. une deuxième structure de réseau ($G_1$) en forme de bande, produisant en tant que réseau de phase un décalage de phase partiel d'un rayonnement X traversant et ayant une deuxième période de réseau ($p_1$),
   1.3. une troisième structure de réseau ($G_2$) en forme de bande avec une troisième période de réseau ($p_2$), utilisée pour détecter un décalage de phase relatif de rayons X voisins ($S_1$, $S_2$) et/ou leurs taux de dispersion,
   1.4. dans lequel les trois structures de réseau ($G_0$, $G_1$, $G_2$) remplissent les conditions de Talbot en ce qui concerne les distances (1, d) entre elles et dans lequel au moins la première et la deuxième structures de réseau ($G_0$, $G_1$) remplissent les conditions de Talbot en ce qui concerne leurs périodes de réseau ($p_0$, $p_1$), et
   1.5. un dispositif pour déterminer en termes de valeur la phase entre rayons X voisins et/ou pour déterminer en termes de valeur l'évolution spatiale de l'intensité par élément détecteur perpendiculairement aux bandes des structures de réseaux,
   **caractérisé en ce que**
   1.6. la troisième structure de réseau ($G_2$) a une période de réseau ($p_2$) plus grande d'un facteur 2 à 5 que la période de réseau ($p_0$) de la première structure de réseau ($G_0$).

2. Système de tomodensitométrie par rayons X (C1) selon la revendication précédente 1, **caractérisé en ce que** ledit au moins un interféromètre à réseau possède une trajectoire du faisceau présentant en direction d'un angle de rotation du portique un évasement ($\alpha$) d'au moins 30°.

3. Système de tomodensitométrie par rayons X (C1) selon l'une des revendications précédentes 1 ou 2, **caractérisé en ce que** ledit au moins un interféromètre à réseau possède une trajectoire du faisceau présentant en direction d'un angle de rotation du portique un évasement ($\alpha$) compris entre 35° et 40°.

4. Système de tomodensitométrie par rayons X (C1) selon l'une des revendications précédentes 1 à 3, **caractérisé en ce que** l'objet à examiner est positionné entre la deuxième structure de réseau ($G_1$) et la troisième structure de réseau ($G_2$).

5. Système de tomodensitométrie par rayons X (C1) selon l'une des revendications précédentes 1 à 4, **caractérisé en ce que** l'extension de la première structure de réseau ($G_0$) en direction circonférentielle du portique est de 1 à 3 cm.

6. Système de tomodensitométrie par rayons X (C1) selon l'une des revendications précédentes 1 à 5, **caractérisé en ce que** l'extension de la troisième structure de réseau ($G_2$) en direction de la plus grande étendue du faisceau utilisé est plus grande au moins d'un facteur 2 que l'extension de la deuxième structure de réseau ($G_1$) en direction de la plus grande étendue du faisceau utilisé.

7. Système de tomodensitométrie par rayons X (C1) selon l'une des revendications précédentes 1 à 6, **caractérisé en ce que** le rapport (l/d) entre la distance (1) séparant la première et deuxième structures de réseau ($G_0$, $G_1$) et la distance séparant la deuxième et troisième structures de réseau ($G_1$, $G_2$) est inférieur à 1.

8. Système de tomodensitométrie par rayons X (C1) selon l'une des revendications précédentes 1 à 6, **caractérisé en ce que** le rapport (l/d) entre la distance (1) séparant la première et deuxième structures de réseau ($G_0$, $G_1$) et la distance séparant la deuxième et troisième structures de réseau ($G_1$, $G_2$) est compris entre les valeurs l/d = 0,5 à 0,1.

9. Système de tomodensitométrie par rayons X (C1) selon l'une des revendications précédentes 1 à 8, **caractérisé en ce que** la première structure de réseau ($G_0$) comprend un réseau de source ($G_0$) avec un foyer (F) disposé en amont en direction du faisceau d'une source de rayons X.

10. Système de tomodensitométrie par rayons X (C1) selon l'une des revendications précédentes 1 à 8, **caractérisé en ce que** la première structure de réseau ($G_0$) est formée par des maxima de rayonnement et des minima de rayonnement émergeant à tour de rôle en forme de bande sur une anode.

**11.** Système de tomodensitométrie par rayons X (C1) selon la revendication précédente 10, **caractérisé en ce que** l'anode est munie d'une surface à structure non homogène, donnant naissance aux maxima de rayonnement et minima de rayonnement émergeant à tour de rôle en forme de bande.

**12.** Système de tomodensitométrie par rayons X (C1) selon la revendication précédente 11, **caractérisé en ce que** la surface de l'anode est munie de reliefs et/ou de creux disposés en forme de bande.

**13.** Système de tomodensitométrie par rayons X (C1) selon la revendication précédente 11, **caractérisé en ce que** la surface de l'anode comprend des matériaux disposés en forme de bande et ayant des numéros atomiques distincts.

**14.** Système de tomodensitométrie par rayons X (C1) selon la revendication précédente 10, **caractérisé en ce qu'**il est prévu un dispositif de déflexion d'un faisceau d'électrons à fonctionnement électromagnétique, qui balaye en forme de bande la surface de l'anode avec le faisceau d'électrons et produit ainsi les maxima de rayonnement et minima de rayonnement émergeant à tour de rôle en forme de bande.

**15.** Système de tomodensitométrie par rayons X (C1) selon l'une des revendications précédentes 1 à 14, **caractérisé en ce que** la troisième structure de réseau $(G_2)$ comprend au moins un réseau d'analyse muni d'un détecteur (D) avec résolution spatiale disposé en aval composé d'une pluralité d'éléments détecteurs $(E_i)$.

**16.** Système de tomodensitométrie par rayons X (C1) selon l'une des revendications précédentes 1 à 15, **caractérisé en ce que** la première structure de réseau $(G_0)$ comprend un dispositif permettant un décalage spatial contrôlé perpendiculairement à ses lignes de réseau et doté d'une résolution spatiale dans le domaine de la période $(p_0)$ de la première structure de réseau $(G_0)$.

**17.** Système de tomodensitométrie par rayons X (C1) selon l'une des revendications précédentes 1 à 15, **caractérisé en ce que** la deuxième structure de réseau $(G_1)$ comprend un dispositif permettant un décalage spatial contrôlé perpendiculairement à ses lignes de réseau et doté d'une résolution spatiale dans le domaine de la période $(p_1)$ de la deuxième structure de réseau $(G_1)$.

**18.** Système de tomodensitométrie par rayons X (C1) selon l'une des revendications précédentes 1 à 15, **caractérisé en ce que** la troisième structure de réseau $(G_2)$ comprend un dispositif permettant un décalage spatial contrôlé perpendiculairement à ses lignes de réseau et doté d'une résolution spatiale dans le domaine de la période $(p_2)$ de la troisième structure de réseau $(G_2)$.

**19.** Système de tomodensitométrie par rayons X (C1) selon l'une des revendications précédentes 1 à 14, **caractérisé en ce que** la troisième structure de réseau $(G_2)$ est formée par une pluralité d'éléments détecteurs $(E_1)$ avec résolution spatiale en forme de bande par rayons X détectée.

FIG 1

FIG 2          Stand der Technik

# FIG 3

FIG 4

$$I(x) = I_{med} + I_{amp}\cos(x+x_0)$$

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1731099 A1
- EP 1803398 A1
- DE 102006017290 A1
- US 5812629 A
- US 7180979 B2
- WO 200712533 A1


**In der Beschreibung aufgeführte Nicht-Patentliteratur**

- **F. PFEIFFER et al.** Hard X-ray dark-field imaging using a grating interferometer. *Nature Materials,* vol. 7, 134-137
- **T. WEITKAMP et al.** Developments in X-Ray Tomography V. *Proc. SPIE 6318,* 2006, 6318-28